# EUROPÄISCHE PATENTSCHRIFT

(11) **EP 1 701 676 B1**
(45) Veröffentlichungstag und Bekanntmachung des Hinweises auf die Patenterteilung: **15.08.2007**
(21) Anmeldenummer: 05700710.6
(22) Anmeldetag: 05.01.2005
(51) Int. Cl.: A61F 13/00, A61F 13/02

(54) **WUNDAUFLAGE**
DRESSING FOR A WOUND
PANSEMENT

(30) Priorität: 09.01.2004 DE 102004001594
(43) Veröffentlichungstag der Anmeldung: 20.09.2006
(73) Patentinhaber: Bio-Gate AG, 90411 Nürnberg (DE)
(72) Erfinder: BECHERT, Thorsten, 91301 Forchheim (DE); STEINRÜCKE, Peter, 91052 Erlangen (DE)
(74) Vertreter: Ehnis, Tobias
(86) Internationale Anmeldenummer: PCT/EP2005/000048
(87) Internationale Veröffentlichungsnummer: WO 2005/065603

(56) Entgegenhaltungen:
- WO-A-00/16913
- WO-A-00/16914
- FR-A- 968 242
- US-A- 4 728 323
- US-A- 5 419 913

## Beschreibung

Die Erfindung betrifft eine Wundauflage.

Es ist allgemein bekannt, dass das Heilen von Wunden durch ein feuchtes Milieu verbessert werden kann. Eine Schorfbildung wird dabei verhindert und es treten seltener Narben auf. Das feuchte Milieu kann durch die Wundheilung unterstützende Medien bewirkt werden. üblicherweise wird dazu ein saugfähiges Material mit einem solchen Medium getränkt und dann auf eine Wunde aufgebracht.

Aus der FR 968242 ist eine Wundauflage bekannt mit einer antimikrobiell wirksamen Substanz, welche mit einem hydrophilen Polymer beschichtet ist.

Aus der DE 297 21 345 U1 ist ein Wundpflaster bekannt, bei welchem zwischen einer Klebefolie und einem Wundvlies eine Flüssigkeitskammer vorgesehen ist. Die Flüssigkeitskammer kann zum Platzen gebracht werden, so dass dadurch darin enthaltene Flüssigkeit das Wundvlies durchdringen kann.

Die DE 196 31 421 A1 offenbart eine antimikrobielle Wundauflage. Die Wundauflage besteht aus einem hydrophoben, Bakterien adsorbierenden Material, das einen antimikrobiellen Wirkstoff enthält, der nicht in die Wunde abgegeben wird. Durch das hydrophobe Material in Kombination mit dem antimikrobiellen Wirkstoff wird erreicht, dass die Bakterien aus der Wundflüssigkeit an der Wundauflage adsorbieren und dort abgetötet werden. Mit dem Entfernen der Wundauflage werden auch die Bakterien entfernt. Sie stören damit nicht mehr den Heilungsverlauf.

Aus der DE 197 27 032 A1 ist ein Pflaster bekannt, bei dem ein haftender Bereich mit einer ersten Klebezone und einer außerhalb der ersten Klebezone angeordneten zweiten Klebezone vorgesehen ist. Die Haftkraft der ersten Klebezone ist geringer als die der zweiten Klebezone.

Aus der DE 198 60 759 C2 ist ein Pflaster bekannt, mittels dem eine Hautfläche abgedeckt werden kann, in die hinein eine Injektion erfolgen soll. Das Pflaster besteht aus einer elastischen Fläche, die hautseitig Klebeflächen aufweist, zwischen denen ein ringförmiger medizinisch wirksamer Träger aus einem saugfähigen Material angeordnet ist. Der Träger enthält ein Desinfektionsmittel zur Desinfektion der vom Träger umschlossenen Injektionszone. Die elastische Fläche ist über der Injektionszone mit einer Öffnung und einer Schutzfolie versehen, die die Klebefläche und den medizinisch wirksamen Träger abdeckt und vor Benutzung entfernt werden muss.

Aus der DE 697 18 035 T2 ist ein Klebeverband mit einer Klebezusammensetzung in Form einer Schicht mit mindestens einem Teil einer anpassbaren Verstärkung bekannt. Die Klebezusammensetzung und die Verstärkung sind so ausgewählt, dass der Klebeverband dampfdurchlässig ist.

Aus der EP 1 005 301 B1 ist ein Wundverband zum Aufkleben auf die Haut bekannt, wobei ein erster Bereich vorgesehen ist, welcher einer Unterlage gegenüber liegt, die für den Kontakt mit einer Wunde vorgesehen ist. Der erste Bereich wird von einem zweiten Bereich umgeben, der einen antimikrobiellen Wirkstoff umfasst, um eine Wanderung von Mikroorganismen in den ersten Bereich von der äußeren Umgebung her zu verhindern.

Aus der WO 02/056927 A2 ist ein mehrschichtiges Verbandsmaterial bekannt. Es enthält eine saugfähige Schicht, eine gasdurchlässige aber flüssigkeitsundurchlässige Außenschicht und in der saugfähigen Schicht eine Kammer zur Abgabe eines flüssigen Behandlungsmediums. Ferner enthält das Verbandsmaterial eine eine Auflage auf der Wunde bildende perforierte Schicht. Die Außenschicht kann mit einer Klebeschicht versehen sein, die ein Fixieren des Verbandsmaterials um die Wunde herum ermöglicht. Durch dieses Verbandsmaterial ist es möglich, eine Wunde okklusiv und feucht zu halten.

Aufgabe der vorliegenden Erfindung ist es, eine Wundauflage bereitzustellen, welche eine noch bessere Wundheilung ermöglicht. Ferner soll ein Verfahren zu deren Herstellung angegeben werden.

Diese Aufgabe wird durch die Merkmale der Ansprüche 1 und 27 gelöst. Vorteilhafte Ausgestaltungen ergeben sich aus den Merkmalen der Ansprüche 2 bis 26.

Erfindungsgemäß ist eine Wundauflage vorgesehen, enthaltend eine durch eine saugfähige Matrix gebildete erste Schicht und eine antimikrobiell wirksame Substanz, wobei die Substanz an einer Oberfläche der Matrix chemisch oder physikalisch gebunden vorliegt. Die Oberfläche der Matrix einschließlich der Substanz ist mit einem hydrophilen Polymer beschichtet. Die Matrix enthält Fasern und kann aus einem Vlies, aus Gaze, einem Schaumstoff oder einem sonstigen weichen saugfähigen Material bestehen. Ein Schaumstoff hat den Vorteil, dass er von der Wunde sich ablösendes Material binden und dadurch von der Wunde fern halten kann, so dass es den Heilungsprozess nicht stört. Die Matrix kann mindestens eine Faser aufweisen oder aus mindestens einer Faser gebildet sein. Die Oberfläche der Matrix, an welche die Substanz gebunden ist, kann dann eine Oberfläche der Faser sein. Die Substanz ist bevorzugt so an der Faser oder Oberfläche der Matrix gebunden, dass sie bei einem bestimmungsgemäßen Gebrauch der Wundauflage nicht oder kaum von der Matrix weggeschwemmt werden kann.

Eine Matrix oder Faser, bei welcher die Substanz an der Oberfläche der Matrix oder Faser gebunden vorliegt, lässt sich z.B. herstellen, indem ein die Matrix oder Faser bildendes Polymer zur Herstellung der Matrix oder Faser mit der Substanz versetzt wird. Bei der resultierenden Matrix oder Faser liegt ein Teil der Substanz an der Oberfläche der Matrix bzw. Faser vor. Ein größerer Teil ist jedoch üblicherweise in der Matrix bzw. Faser so vom Polymer eingebettet, dass er von außen nicht für Flüssigkeit zugänglich ist. Vorzugsweise ist die Substanz daher ausschließlich an der Oberfläche der Matrix oder Faser gebunden. Dadurch muss bei der Herstellung keine unnötige Menge der Substanz eingesetzt werden, welche letztendlich unzugänglich ist.

Voraussetzung für eine gute Wundheilung ist, dass es in der Matrix und einer die Matrix umgebenden Flüssigkeit nicht zu einer Vermehrung unerwünschter Keime kommt. Um das zu verhindern, enthält die Wundauflage die antimikrobiell wirksame Substanz. Eine Substanz ist antimikrobiell wirksam, wenn sie, gemessen wie beschrieben in der DE 197 58 598 A1, die Vermehrung von Keimen signifikant verzögert oder komplett unterbindet. Eine antimikrobiell wirksame Substanz ist auch eine Substanz, die durch Umwandlung einen antimikrobiellen Wirkstoff in der Umgebung hervorbringt, in der die Wundauflage bestimmungsgemäß verwendet werden soll. Ist beispielsweise der antimikrobielle Wirkstoff ein Metallion oder ein das Metallion enthaltender ionischer Komplex, so sind auch das Metall, dessen Legierungen und andere Substanzen antimikrobiell wirksam, aus denen das Metallion oder der das Metallion enthaltende ionische Komplex im Bereich einer Wunde freigesetzt werden kann. Bei dem Metallion kann es sich um ein Silber-, Kupfer-oder Zink-Kation handeln. Die antimikrobiell wirksame Substanz kann dann metallisches Silber, Kupfer bzw. Zink oder eine Legierung oder eine andere Substanz sein, aus der das genannte Ion im Bereich einer Wunde freigesetzt werden kann.

Die antimikrobiell wirksame Substanz kann gegenüber Staphylococcus *epidermidis* oder anderen Mikroorganismen antimikrobiell wirksam sein. Die antimikrobielle Wirksamkeit der Substanz im Hinblick auf andere Mikroorganismen wird entsprechend dem aus der DE 197 58 598 A1 bekannten Verfahren mit dem jeweils zu untersuchenden Mikroorganismus anstelle von *Staphylococcus epidermidis* ermittelt. Besonders bevorzugt sind solche Substanzen, die antimikrobiell wirksam sind gegen einen oder mehrere der Mikroorganismen der Gruppe Bacillus, *Clostridium, Enterobacter, Escherichia*, *Pseudomonas, Salmonella, Staphylococcus, Yersinia, Candida* und *Aspergillus.*

Es ist festgestellt worden, dass eine antimikrobiell wirksame Substanz, insbesondere wenn sie zytotoxisch ist, die Wundheilung stören kann. Die antimikrobiell wirksame Substanz ist zytotoxisch, wenn sie eine zytotoxische Wirkung wie in DIN-ISO 10993-5 beschrieben aufweist. Um eine Störung zu verhindern, ist es erfindungsgemäß vorgesehen, dass die Substanz an der Oberfläche der Matrix bzw. der Fasern der Matrix gebunden ist. Durch die Bindung kann verhindert werden, dass die antimikrobiell wirksame Substanz in einer eine Wundheilung störenden Menge in die Wunde gerät. Weiterhin kann dadurch verhindert werden, dass die antimikrobiell wirksame Substanz durch Aufnahme in den Körper aus dem Bereich der Wundauflage verloren geht und dadurch dort nicht mehr langfristig antimikrobiell wirksam sein kann. Weiterhin kann dadurch verhindert werden, dass die Substanz nach einer Aufnahme in den Körper unerwünschte, z.B. allergische Reaktionen, auslöst.

Durch die erfindungsgemäße Wundauflage kann es verhindert werden, dass sich, beispielsweise aus der Wunde stammende, Keime in der Wundauflage vermehren und dann zu einer Infektion bzw. Reinfektion der Wunde führen. Durch das langfristige Verhindern des Keimwachstums kann die Wundauflage bis zum Abheilen der Wunde auf der Wunde belassen werden. Dadurch kann eine mechanische Belastung der Wunde durch ein Entfernen der Wundauflage vermieden und eine für die Wundheilung förderliche Wundruhe gewährleistet werden. Der wesentliche Vorteil der erfindungsgemäßen Wundauflage besteht darin, dass die Wundauflage selbst keimfrei bleibt, während der Wundheilungsprozess nicht durch die antimikrobiell wirksame Substanz gestört wird.

Die Beschichtung aus dem Polymer ist so dünn ausgebildet und das Polymer so gewählt, dass die Wirkung der Substanz dadurch nicht verhindert wird. Ein solches Polymer kann z. B. ein durch Plasmapolymerisation aus Hexamethyldisiloxan gebildetes Polymer sein. Die Beschichtung verbessert die Bindung der Substanz auf der Faser oder der Matrix. Beispielsweise werden durch Verdampfen und Abscheiden aufgetragene Cluster aus Silber vor einem mechanischen Abrieb geschützt. Durch die Hydrophilie der Schicht wird die Benetzbarkeit der Faser bzw. der Matrix begünstigt. Dadurch wird eine gleichmäßigere Verteilung der Flüssigkeit über die Matrix erreicht. Weiterhin kann durch den verbesserten Kontakt der Faser bzw. der Matrix mit der Flüssigkeit auch eine bessere Wirkung der antimikrobiell wirksamen Substanz erreicht werden. Darüber hinaus kann durch das, insbesondere durch Plasmapolymerisation gebildete, hydrophile Polymer eine Adhäsion von grampositiven und/oder gramnegativen Keimen auf der Faser bzw. der Matrix vermindert werden. Ferner verbessert die hydrophile Schicht das Gleitverhalten der Matrix oder einzelner Fasern der Matrix im feuchten Milieu, weil sich direkt an der Oberfläche der beschichteten Matrix oder Faser ein Flüssigkeitsfilm ausbildet. Liegt die Matrix dann auf der Wunde auf, gleitet sie bei einer Verschiebung leicht über die Wunde und übt dabei nur eine geringe mechanische Belastung auf die Wunde aus. Dadurch wird bei einem verminderten Flüssigkeitspolster zwischen der Matrix und der Wunde der Tragekomfort der Wundauflage deutlich erhöht. Darüber hinaus kann der Flüssigkeitsfilm auf der beschichteten Faser oder Matrix auch das Einwachsen des sich neu in der Wunde bildenden Gewebes in die Matrix verhindern.

Vorzugsweise weist die Wundauflage eine mit der Matrix verbundene durch eine gasdurchlässige aber flüssigkeitsundurchlässige Folie gebildete zweite Schicht und einen selbstklebenden ersten Bereich auf. Die Matrix ist mit der Folie in einem zweiten Bereich verbunden. Die Matrix kann mit der Folie beispielsweise dadurch verbunden sein, dass sie mit der Folie verklebt oder verschweißt ist. Der selbstklebende erste Bereich ist ein Bereich der Folie, der den zweiten Bereich, vorzugsweise beabstandet, umgibt, wobei die Wundauflage mittels des selbstklebenden ersten Bereichs auf eine menschliche oder tierische Haut geklebt werden kann. Durch das Aufkleben wird ein die Matrix enthaltender mit einer Flüssigkeit füllbarer flüssigkeitsdichter Innenraum gebildet. Der Innenraum ermöglicht eine flüssige Wundversorgung.

Dieser Ausführungsform der Erfindung liegt die Erkenntnis zu Grunde, dass bei einer üblichen feuchten Wundbehandlung das saugfähige Material in einer fortgeschrittenen Phase der Wundheilung zu trocknen beginnt und der Wunde dadurch Feuchtigkeit entzieht. Dadurch wird der Wundheilungsverlauf verschlechtert.

Weiterhin liegt dieser Ausführungsform der Erfindung die Erkenntnis zu Grunde, dass die Wundheilung noch dadurch verbessert werden kann, dass die Wunde nicht nur feucht gehalten wird, sondern mit so viel Flüssigkeit versorgt wird, dass sich ein Flüssigkeitspolster über der Wunde bildet. Dadurch wird die Wundheilung verbessert und die Tendenz zur Narbenbildung verringert. Eine solche Wundversorgung wird hier als flüssige Wundversorgung bzw. Liquid-Wundversorgung bezeichnet. Mit der erfindungsgemäßen Wundauflage kann das erreicht werden, indem die Wundauflage zunächst trocken auf die zu behandelnde Wunde aufgelegt und mittels des selbstklebenden ersten Bereichs an der die Wunde umgebenden Haut rundum festgeklebt wird, so dass der Wundbereich dadurch flüssigkeitsdicht verschlossen wird. Anschließend kann mittels einer Kanüle die Folie durchstochen und eine Flüssigkeit in den darunterliegenden Innenraum injiziert werden. Nach dem Herausziehen der Kanüle kann die entstandene Öffnung in der Folie mittels einer Klebefolie verschlossen werden. Vorteilhafterweise wird die Flüssigkeit mehrfach, z.B. einmal pro Tag, zugeführt. Dadurch kann sichergestellt werden, dass die Wunde bis zur Heilung und zum Entfernen der Wundauflage permanent unter einem Flüssigkeitspolster gehalten wird.

Bevorzugt ist der selbstklebende erste Bereich mit einem Klebstoff versehen, der gut hautverträglich ist und sich durch eine wässrige Flüssigkeit nicht löst. Solche Klebstoffe sind im Stand der Technik, insbesondere im Bereich der Pflaster, bekannt. Weiterhin sollte der Klebstoff auch einem erhöhten Flüssigkeitsdruck im Innenraum standhalten, ohne sich von der Haut zu lösen. Ein erhöhter Flüssigkeitsdruck kann bereits durch das Injizieren der Flüssigkeit und eine von der Folie ausgeübte Spannung erzeugt werden. Ein erhöhter Flüssigkeitsdruck kann beispielsweise auch durch eine mechanische Belastung der Wundauflage durch den Patienten, z.B. durch ein Bewegen der die Wunde aufweisenden Körperpartie, bewirkt werden.

Dadurch, dass die Matrix mit der Folie verbunden ist, kann die Matrix mittels dem Flüssigkeitspolster von der Wunde abgehoben werden. Die Wunde steht dadurch nicht direkt mit der Matrix und der antimikrobiell wirksamen Substanz in Kontakt. Durch das Abheben der Matrix von der Wunde kann auch ein unerwünschtes Einwachsen von in der Wunde neu gebildetem Gewebe in die Matrix vermieden werden. Eine eine Auflage auf der Wunde bildende und die Wunde von der Matrix trennende perforierte Schicht, wie sie aus der WO 02/056927 A2 bekannt ist, ist daher nicht erforderlich. Die Matrix dient hier als Träger der Substanz und nicht dazu, die Wunde feucht zu halten, wie das bei den bekannten Wundauflagen der Fall ist. Das Abheben der Matrix von der Wunde kann dadurch begünstigt werden, dass der selbstklebende erste Bereich beabstandet zu einem zweiten Bereich angeordnet ist, an dem die Matrix mit der Folie verbunden ist.

Eine Antisepsis der Wunde kann durch eine entsprechende Wahl der Flüssigkeit erreicht werden. Beispielsweise kann der pH-Wert der Flüssigkeit so niedrig sein, dass dadurch ein Wachstum von Keimen vermindert wird. Eine Flüssigkeit mit einem geringen pH-Wert hat außerdem eine adstringierende Wirkung auf die Wunde, wodurch der Heilungsprozess begünstigt werden kann. Weiterhin kann die Lösung das Zellwachstum stimulierende Faktoren und/oder Nährstoffe enthalten.

Vorzugsweise ist die Substanz eine anorganische Substanz, insbesondere ein Metall oder eine Metallverbindung. Eine solche antimikrobiell wirksame Substanz ist gewöhnlich preiswert, leicht erhältlich und leicht zu verarbeiten. Unter einer Metallverbindung wird hier eine Mischung oder eine Legierung aus mindestens zwei Metallen verstanden. Von dem Metall oder der Metallverbindung können als Wirkstoffe Metallionen oder Metallionen enthaltende Komplexe gebildet und freigesetzt werden. Bevorzugt handelt es sich um ein oligodynamisch, d.h. in kleinsten Mengen, antimikrobiell wirksames Metall bzw. eine oligodynamisch antimikrobiell wirksame Metallverbindung.

In einer bevorzugten Ausgestaltung ist die Substanz ausgewählt aus einer Gruppe bestehend aus Silber, Kupfer und Zink, deren Ionen und deren Metallkomplexe oder einer Mischung oder Legierung umfassend mindestens eine dieser Komponenten. Die Legierung kann neben den genannten Metallen insbesondere auch Gold und/oder Platin enthalten. Eine solche Substanz wirkt gegen eine Vielzahl verschiedener Mikroorganismen und greift auf zahlreiche Weisen in deren Stoffwechsel ein. Dementsprechend kommt es bei Verwendung einer solchen Substanz seltener zur Resistenzbildung bei Bakterien als bei Verwendung spezifisch wirkender organischer antimikrobieller Substanzen, wie Antibiotika. Vorzugsweise ist die Substanz Silber, ein Silber-Kation oder ein Silber- bzw. Silberkation-freisetzender Komplex oder eine Silber- bzw. Silberkation-freisetzende Legierung. Insbesondere metallisches Silber ist leicht verarbeitbar und in hoher Qualität zu einem verhältnismäßig geringem Preis erhältlich, so dass auch die erfindungsgemäße Wundauflage wiederum verhältnismäßig preiswert hergestellt werden kann.

Das Metall oder die Metallverbindung kann, insbesondere durch Verdampfen und Abscheiden, durch einen Sputterprozess oder durch Chemical Vapor Deposition, in Form von Clustern auf die Faser oder der Oberfläche der Matrix aufgebracht sein. Beim Verdampfen und Abscheiden wird das Metall oder die Metallverbindung in einem Vakuum thermisch verdampft und der Metalldampf anschließend auf der Faser oder der Matrix abgeschieden. Das Metall oder die Metallverbindung liegt durch das Aufbringen mittels Verdampfen und Abscheiden, des Sputterprozesses oder Chemical Vapor Deposition auf der Faser oder der Matrix in Form von Clustern vor. Die Cluster weisen besonders gute antimikrobielle Eigenschaften auf.

Zweckmäßigerweise liegt die Substanz in der erfindungsgemäßen Wundauflage in körniger Form vor, wobei eine mittlere Korn- bzw. Partikelgröße von 5 bis 100 nm bevorzugt ist. Die Substanz kann in Form von einzelnen oder untereinander verketteten Partikeln vorliegen. Solche feinen Pulver antimikrobiell wirksamer Substanzen lassen sich insbesondere für anorganische Substanzen, und hierbei insbesondere für Silber, aber auch für Kupfer und Zink, sowie Mischungen, Komplexe und Legierungen der drei genannten Metalle leicht herstellen. Aufgrund der geringen mittleren Korngröße besitzt die Substanz eine hohe spezifische Oberfläche, so dass sie insbesondere durch Diffusion gut von der Matrix abgegeben werden kann. Ferner ist es vorteilhaft, dass aufgrund der hohen spezifischen Oberfläche eine chemische Inaktivierung des körnigen Wirkstoffs, wie sie in einer Wundumgebungen gelegentlich vorkommen kann, gewöhnlich nur einen Teil der Oberfläche betrifft, so dass eine Abgabe der Substanz von der Matrix auch unter widrigen Bedingen ermöglicht wird. Als besonders vorteilhaft hat sich eine mittlere Korngröße der Substanz von 5 bis 50 nm, vorzugsweise 5 bis 20 nm erwiesen. Wenn die Substanz Silber oder eine Silber-Legierung ist, so spricht man bei diesen Korngrößenverteilungen auch von nanoskaligem (nano scale) Silber bzw. einer nanoskaligen Silber-Legierung.

Die Substanz kann in einer Schichtdicke von zumindest 1 nm, und vorzugsweise nicht mehr als 1 mm vorliegen. Bei Verwendung körniger Substanzen ist die Schicht zumindest so dick wie der körnige Wirkstoff. Vorzugsweise beträgt die mittlere Schichtdicke zumindest 5 nm bis 100 nm, wobei besonders Schichtdicken von 10 nm bis 50 nm bevorzugt werden, insbesondere wenn die Substanz Silber, Kupfer und/oder Zink bzw. deren Ionen, Metallkomplexe oder eine Mischung oder Legierung dieser Elemente ist. Es hat sich gezeigt, dass bereits derartig geringe Schichtdicken einer antimikrobiellen, insbesondere nanoskaliges Silber enthaltenden, Substanz ausreichend sind, um eine antimikrobielle aber nicht cytotoxische Wirkung erreichen zu können.

Die Substanz liegt vorzugsweise in einer solchen Menge vor, dass sie bei einem Durchnässen der Matrix mit einer Flüssigkeit in der Matrix, insbesondere in der gesamten Matrix, antimikrobiell wirkt. Eine solche Menge lässt sich mittels einfacher Routineversuche ermitteln. Handelt es sich bei der Substanz um metallisches Silber, kann eine ausreichende antimikrobielle Wirkung in der Matrix durch einen Silbergehalt der Matrix von 1 µg bis 200 µg pro cm² einer von der Matrix maximal bedeckbaren Fläche erreicht werden. Höhere Silbergehalte sind unvorteilhaft, weil dadurch Silberionen in einer solchen Menge freigesetzt werden können, dass diese sich negativ auf die Wundheilung auswirken.

Bevorzugt ist das Polymer ein die Adhäsion von Bakterien, vorzugsweise gramnegativen Bakterien oder Staphylokokken, insbesondere Staphylokokkus epidermidis, auf der Faser oder Matrix verminderndes Polymer. Neben der Substanz bewirkt auch diese Maßnahme eine verminderte Besiedlung der Matrix mit Keimen.

Vorzugsweise ist die Faser oder Oberfläche der Matrix mittels Plasmapolymerisation mit dem Polymer beschichtet. Dadurch wird eine extrem dünne Ausgestaltung der Schicht ermöglicht, welche die Wirkung der Substanz kaum beeinträchtigt. Durch die Wahl der Parameter bei der Plasmapolymerisation können die Eigenschaften des Polymers beeinflusst werden. Der Fachmann kann unter Durchführung üblicher Routineversuche geeignete Ausgangsmaterialien und Parameter für die Herstellung einer entsprechenden Polymerschicht ermitteln. Die Beschichtung der Matrix mit der antimikrobiellen Substanz und die Plasmapolymerisation kann beispielsweise wie folgt durchgeführt werden:

Auf eine aus Vlies bestehende Matrix werden in einem ersten Schritt Cluster nanoskaligen Silbers aufgetragen. Hierzu wird unter einer Schutzgasatmosphäre von z.B. Argon bei etwa 10 mbar Arbeitsdruck metallisches Silber verdampft. Dabei wird auf Fasern der Matrix Silber in Form von einzelnen oder untereinander verketteten Silberpartikeln abgelagert. Die mittlere Partikelgröße der Silberpartikel beträgt etwa 10 bis 20 nm. Das Silber ist in einer Dicke von etwa 20 nm aufgetragen. In einem zweiten Schritt wird eine Plasmapolymerschicht mit Hexamethyldisiloxan (HMDSO) als Monomer bzw. Precursor aufgebracht. Die Plasmapolymerisation wird bei einem Arbeitsdruck von 0,07 mbar mit einem Arbeitsgas aus 95 % O₂ und 5 % HMDSO durchgeführt. Nach 45 Sekunden der so durchgeführten Plasmapolymerisation ist das aufgetragene Silber mit einer 45 nm dicken und stark hydrophilen Plasmapolymerschicht versehen.

Die Oberflächenenergie der Beschichtung beträgt dabei 105 mN/m.

Das Auftragen des Polymers auf die Faser kann vor einem Herstellen der Matrix aus der Faser oder danach erfolgen. Erfolgt es danach, wird die gesamte Matrix, beispielsweise ein Vlies oder ein Textilgewebe, einem Beschichtungsverfahren, wie der Plasmapolymerisation, unterzogen. Besonders vorteilhaft ist es, wenn das Polymer nach der Plasmapolymerisation oxidiert wird. Dadurch kann eine extrem hydrophile Oberfläche geschaffen werden.

In einer bevorzugten Ausgestaltung ist das Polymer aus Monomeren auf Acrylsäurebasis oder aus Monomeren auf Siloxanbasis, insbesondere Hexamethyldisiloxan, gebildet. Ein solches Polymer kann antiadhäsive Eigenschaften gegenüber Bakterien mit guten hydrophilen Eigenschaften vereinigen. Ferner erlaubt es eine gute Wirkung einer vom Polymer auf der Matrix abgedeckten Substanz, wie beispielsweise metallischem Silber.

Das Polymer liegt vorzugsweise in einer Schicht mit einer mittleren Dicke von 5 nm bis 500 nm vor. Insbesondere bei einem plasmapolymerisierten Polymer ist es jedoch bevorzugt, wenn die Dicke 5 bis 200 nm, vorzugsweise 10 bis 100 nm beträgt. Bei diesen Schichtdicken lassen sich insbesondere mit durch Plasmapolymerisation hergestellten Polymerschichten hervorragende antimikrobielle und nicht cytotoxische Beschichtungen herstellen. Gleichzeitig sind diese Beschichtungen sehr dünn, so dass sie optisch kaum auffallen oder sogar transparent sein können.

Vorzugsweise ist die Menge, in welcher die Substanz vorliegt, so bemessen, dass die Menge von der Substanz gebildeter und/oder freigesetzter Wirkstoffe auf eine Wunde im Anwendungsfall nicht zytotoxisch wirkt. Eine solche Menge lässt sich mittels einfacher Routineversuche ermitteln. Bei den Wirkstoffen kann es sich z.B. um Metallionen oder um Komplexe dieser Metallionen handeln. Ist die Substanz Silber, ist eine geeignete Menge Silber in der Matrix beispielsweise 1 µg bis 200 µg, vorzugsweise 5µg bis 35 µg, insbesondere 5 µg bis 15 µg, pro cm² einer von der Matrix maximal bedeckbaren Fläche. Es ist bisher nicht erkannt worden, dass Silberionen durch die üblicherweise verwendeten Silber enthaltenden Wundauflagen in Mengen freigesetzt werden könnten, die zytotoxisch auf Wunden wirken. Dadurch kann die Wundheilung gestört werden.

Bevorzugt sind an der Matrix eine Wundheilung unterstützende Substanzen, insbesondere Wachstumsfaktoren, gebunden. Solche Substanzen können beispielsweise der epidermale Wachstumsfaktor (EGF), der Plättchenwachstumsfaktor (PDGF), der Vaskuläre Endotheliale Wachstumsfaktor (VEGF) oder der Keratinozytenwachstumsfaktor (KGF) sein. Vorzugsweise ist die Matrix mit einer die Wundheilung unterstützenden, insbesondere sauren oder die Wundheilung unterstützende Nährstoffe enthaltenden, Flüssigkeit durchnäßt oder getränkt.

In einer besonders bevorzugten Ausgestaltung ist die Folie, zumindest stellenweise, durchsichtig. Dadurch kann der Flüssigkeitsstand unter der Folie beobachtet werden und sobald ein verminderter Flüssigkeitsgehalt im Innenraum beobachtet wird, neue Flüssigkeit nachgefüllt werden.

Bei einer weiteren besonders vorteilhaften Ausgestaltung der Erfindung ist die gesamte Wundauflage, einschließlich Matrix und Folie, für Licht, insbesondere UV-Licht, Infrarot-Licht (IR-Licht) oder Nahinfrarotes Licht (NIR-Licht), durchlässig.

Das kann durch die Wahl eines entsprechenden Materials erreicht werden. Dadurch ist es möglich, die Wunde einer Fototherapie zu unterziehen ohne die Wundauflage zu entfernen. Eine Fototherapie kann die Wundheilung erheblich beschleunigen.

Vorzugsweise enthält die Wundauflage einen Indikator. Der Indikator kann ein Farbstoff sein, der einen bestimmten Zustand der Wundauflage oder der Wunde anzeigt. Beispielsweise kann es sich dabei um einen pH-Indikator handeln, der in Abhängigkeit vom pH-Wert seine Farbe ändern kann. Die Farbänderung kann durch die zumindest stellenweise durchsichtige Folie beobachtet werden. Der Indikator kann auch von einer Mehrzahl von Substanzen gebildet werden, die zusammenwirken, um einen bestimmten Zustand anzuzeigen. Der Indikator kann auch ein Sensor sein. Der Sensor kann z. B. ein leitfähiges Polymer sein, das je nach Zustand der Wunde oder Wundauflage seine Leitfähigkeit ändert. Der Sensor kann auch ein Biosensor sein. Unter einem Biosensor ist ein Messfühler zu verstehen, bei dem Biomoleküle an Transduktoren, wie z. B. potenziometrische Sensoren, gekoppelt sind. Die Transduktoren wandeln ein beim Binden eines spezifischen Stoffs an das Biomolekül entstehendes Signal in ein elektrisches Signal um. Der Sensor kann Bestandteil eines mehrere Sensoren enthaltenden Sensorfelds oder Biosensorfelds sein. Durch unterschiedliche Sensoren können gleichzeitig mehrere den Zustand der Wunde oder der Wundauflage charakterisierende Parameter bestimmt werden.

Es kann sich beim Indikator auch um einen Indikator handeln, der den Flüssigkeitsgehalt der Matrix anzeigen kann. Dadurch kann ein Trocknen der Matrix besser und frühzeitiger erkannt werden. Der Indikator kann auch ein Indikator sein, der den Grad und/oder Typ einer mikrobiellen Kontamination der Matrix oder der Wunde anzeigen kann. Das kann z. B. durch einen immunologischen Indikator erreicht werden. Dabei können beispielsweise Mikroorganismen oder Bestandteile von Mikroorganismen von einem Antikörper spezifisch gebunden werden und dadurch eine Farbreaktion auslösen. Der Indikator kann auch ein Indikator sein, der einen Entzündungsstatus der Wunde anzeigen kann. Ein solcher Indikator kann ebenfalls ein immunologischer Indikator sein. Dabei können Entzündungsfaktoren von einem Antikörper spezifisch gebunden werden und dadurch eine Farbreaktion auslösen.

Weiterhin betrifft die Erfindung ein Verfahren zur Herstellung einer erfindungsgemäßen Wundauflage, mit folgenden Schritten:
- Bereitstellen einer saugfähigen Matrix,
- Bereitstellen einer gasdurchlässigen aber flüssigkeitsundurchlässigen Folie,
- Anbringen eines selbstklebenden ersten Bereichs (16) auf oder an der Folie und
- Verbinden der saugfähigen Matrix mit der Folie in einem zweiten Bereich der Folie,
wobei der erste Bereich den zweiten Bereich der Folie umgibt,
wobei durch Verdampfen und Abscheiden, einen Sputterprozess oder Chemical Vapor Deposition zuerst eine antimikrobiell wirksame Substanz und dann durch Plasmapolymerisation ein Polymer auf der Matrix oder einer die Matrix bildenden Faser abgeschieden wird.

Die Matrix kann ein Vlies, eine Gaze, ein Schaumstoff oder ein sonstiges weiches saugfähiges Material sein. Die Substanz und das Polymer können auf der fertig gestellten Matrix abgeschieden werden. Die die Matrix bildende Faser muss keine Faser sein, welche ausschließlich die Matrix bildet. Die die Matrix bildende Faser kann eine Faser sein, die bei oder nach der Herstellung der Matrix in die Matrix eingearbeitet wird. Durch das Verdampfen und Abscheiden, den Sputterprozess oder die Chemical Vapor Deposition in Kombination mit der Plasmapolymerisation ist es möglich, eine besonders dünne und gleichzeitig antimikrobiell hoch wirksame Auftragung der Substanz und eine besonders dünne spezifische Eigenschaften aufweisende Auftragung des Polymers auf der Faser bzw. Matrix zu erreichen. Es wird nur wenig Substanz und/oder Polymer benötigt. Die Substanz wird durch das Polymer, insbesondere vor mechanischem Abrieb, geschützt.

Nachfolgend wird ein Ausführungsbeispiel der Erfindung anhand der Zeichnungen näher erläutert. Es zeigen:
- Fig. 1: eine schematische Darstellung der erfindungsgemäßen Wundauflage in seitlicher Ansicht,
- Fig. 2: eine schematische Darstellung der erfindungsgemäßen Wundauflage in einer Draufsicht und
- Fig. 3: eine Anordnung grafischer Darstellungen des zeitlichen Verlaufs des in Form von optischer Dichte (OD) eines Mediums gemessenen Wachstums von Bakterien in Kontakt mit einer herkömmlichen Matrix und einer Matrix einer erfindungsgemäßen Wundauflage.

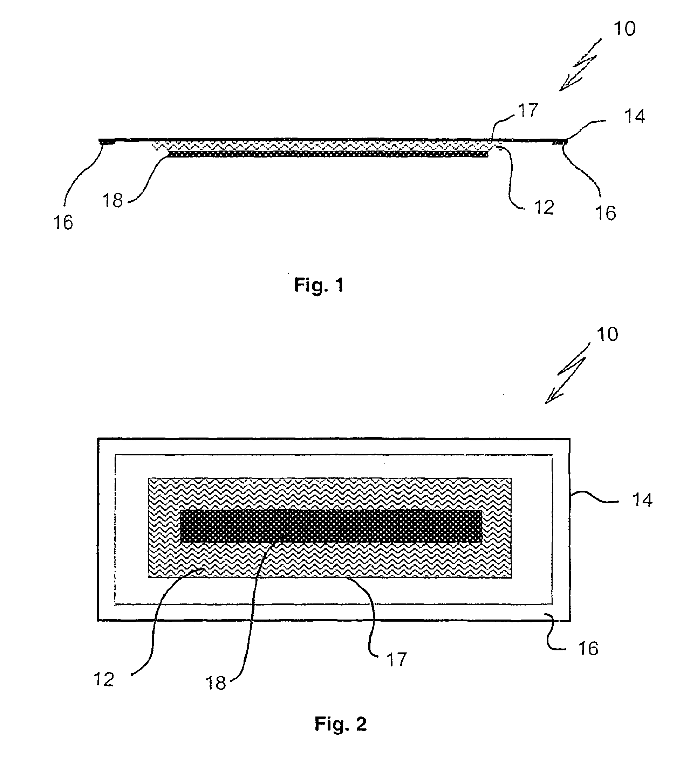

Fig. 1 und 2 zeigen eine erfindungsgemäße Wundauflage 10 mit einer saugfähigen Matrix 12, einer gasdurchlässigen aber flüssigkeitsundurchlässigen Folie 14 und einem selbstklebenden ersten Bereich 16. Der erste Bereich 16 ist hier ein Bereich der Folie, auf dem ein Klebstoff aufgetragen ist. Der erste Bereich 16 umgibt hier einen die Matrix kontaktierenden zweiten Bereich 17 der Folie. Beim bestimmungsgemäßen Gebrauch der Wundauflage 10 wird die Matrix 12 auf die Wunde 18 aufgelegt und der selbstklebende erste Bereich 16 rundum mit der die Wunde 18 umgebenden Haut verklebt. Anschließend kann durch die Folie 14 hindurch eine Flüssigkeit in den durch das Verkleben gebildeten Innenraum injiziert werden. Das beim Injizieren in der Folie 14 entstandene Loch wird mittels einer Klebefolie verschlossen.

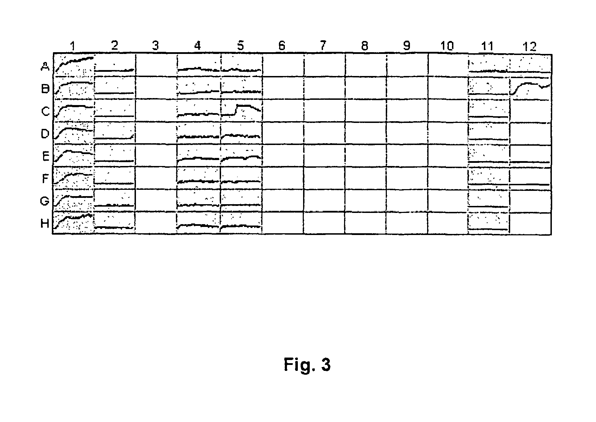

In einer Anwendungsstudie wurde bei einer ersten Patientengruppe eine erfindungsgemäße Wundauflage mit einer Matrix eingesetzt, deren Fasern mit Silber und einem durch Plasmapolymerisation aus Hexamethyldisiloxan gebildeten hydrophilen Polymer beschichtet worden waren. Die Wundauflage wurde dabei für eine postoperative flüssige Wundversorgung beim klinischen Bild des Pectus excavatum (Trichterbrust) über mindestens 4 Tage eingesetzt. Bei einer als Kontrolle dienenden zweiten Patientengruppe wurde eine herkömmliche Wundversorgung durchgeführt. Während es in der zweiten Gruppe gelegentlich zu Infektionen kam, wurden in der ersten Gruppe keine Infektionen beobachtet. Darüber hinaus war der Tragekomfort der erfindungsgemäßen Wundauflage durch die hydrophile Beschichtung deutlich besser als bei der zur herkömmlichen Wundversorgung eingesetzten Wundauflage. Die flüssige Wundversorgung mit der erfindungsgemäßen Wundauflage ermöglichte eine schnellere Heilung bei einer geringeren Tendenz zur Narbenbildung. Die Matrices der zur Wundversorgung eingesetzten Wundauflagen waren auch nach 4 Tagen auf einer Wunde noch antibakteriell wirksam, wie in dem in Fig. 3 dargestellten Versuch gezeigt worden ist.

Die in Fig. 3 gezeigten Ergebnisse sind nach dem aus der DE 197 58 598 A1 bekannten Verfahren ermittelt worden. Dieses Verfahren ist ferner beschrieben in Bechert, Thorsten et al., Nature Medicine (2000), Bd. 6, Nr. 8, Seiten 1053 bis 1056. Der Offenbarungsgehalt der beiden vorgenannten Dokumente wird hier einbezogen. Die zu testenden Matrices wurden in dem Test wie beschrieben eingesetzt.

In Fig. 3 zeigt jedes Feld eine x-y-Grafik, bei der auf der x-Achse die Zeit und auf der y-Achse die optische Dichte aufgetragen ist. Die in den Spalten 1, 2, 4, 5, 11 und 12 der Fig. 3 dargestellten Versuchsergebnisse sind in parallelen, den Reihen A bis H entsprechenden Versuchsansätzen A bis H mit folgenden Matrices ermittelt worden:

| | |
|---|---|
| Spalte 1, Reihen A-H: | Matrix unbeschichtet, |
| Spalte 2, Reihen A-H: | Matrix beschichtet mit Silber und einem durch Plasmapolymerisation aus Hexamethyldisiloxan gebildeten Polymer, |
| Spalte 4, Reihen A-H: | Matrix beschichtet mit Silber und einem durch Plasmapolymerisation aus Hexamethyldisiloxan gebildeten Polymer aus einer 4 Tage lang in der obigen Anwendungsstudie benutzten ersten Wundauflage, |
| Spalte 5, Reihen A-H: | Matrix beschichtet mit Silber und einem durch Plasmapolymerisation aus Hexamethyldisiloxan gebildeten Polymer aus einer 4 Tage lang in der obigen Anwendungsstudie benutzten zweiten Wundauflage, |
| Spalte 11, Reihen A-H: | Sterilkontrollen |
| Spalte 12, Reihe A: | Positivkontrolle |
| Spalte 12, Reihe B: | Negativkontrolle |
| Spalte 12, Reihe E, F: | Leerwert |

Bei den Sterilkontrollen wurde jeweils nur Medium ohne Zusatz von Staphylococcus epidermidis eingesetzt, um zu zeigen, dass das Bakterienwachstum nicht vom Medium herrührt. Bei der Positivkontrolle wurde ein metallisches Silber enthaltendes Polymer eingesetzt. Die Werte zeigen, dass die eingesetzten Bakterien gegenüber Silber sensitiv sind und davon abgetötet werden können. Bei der Negativkontrolle wurde das gleiche Polymer eingesetzt, dass jedoch kein Silber enthielt. Beim Leerwert handelt es sich um einen in einer leeren Vertiefung der Mikrotiterplatte gemessenen Wert, der bei einer Auswertung von allen Messwerten zu subtrahieren ist.

Die Versuchsergebnisse zeigen, dass die mit Silber und dem durch Plasmapolymerisation aus Hexamethyldisiloxan gebildeten Polymer beschichtete Matrix der erfindungsgemäßen Wundauflage hoch antibakteriell bzw. bakterizid wirkt. Diese Wirkung ist auch nach 4 Tagen auf einer Wunde noch vorhanden.

## Patentansprüche

1. Wundauflage (10), enthaltend eine durch eine saugfähige Matrix (12) gebildete erste Schicht und eine antimikrobiell wirksame Substanz, wobei die Substanz an einer Oberfläche der Matrix (12) chemisch oder physikalisch gebunden vorliegt, wobei die Oberfläche der Matrix einschließlich der Substanz mit einem hydrophilen Polymer beschichtet ist, wobei die Wundauflage (10) eine mit der Matrix (12) verbundene durch eine gasdurchlässige aber flüssigkeitsundurchlässige Folie (14) gebildete zweite Schicht enthält und einen selbstklebenden ersten Bereich (16) aufweist, wobei die Matrix (12) mit der Folie (14) in einem zweiten Bereich (17) verbunden ist und der erste Bereich ein Bereich der Folie (14) ist, der den zweiten Bereich (17) umgibt, wobei die Wundauflage (10) mittels des selbstklebenden ersten Bereichs auf eine menschliche oder tierische Haut geklebt werden kann, wobei ein die Matrix (12) enthaltender mit einer Flüssigkeit füllbarer flüssigkeitsdichter Innenraum gebildet wird, wobei die Substanz ein Metall oder eine Metallverbindung ist.

2. Wundauflage (10) nach Anspruch 1, wobei die Matrix (12) mindestens eine Faser aufweist oder aus mindestens einer Faser gebildet ist und die Oberfläche der Matrix (12), an welche die Substanz gebunden ist, eine Oberfläche der Faser ist.

3. Wundauflage (10) nach einem der vorhergehenden Ansprüche, wobei die Substanz ausschließlich an der Oberfläche der Matrix oder Faser gebunden ist.

4. Wundauflage (10) nach einem der vorhergehenden Ansprüche, wobei die Substanz ausgewählt ist aus einer Gruppe bestehend aus Silber, Kupfer und Zink, oder einer Mischung aus Silber, Kupfer und Zink, oder einer Mischung oder Legierung umfassend mindestens eine dieser Komponenten.

5. Wundauflage (10) nach einem der vorhergehenden Ansprüche, wobei das Metall oder die Metallverbindung in Form von, insbesondere durch Verdampfen und Abscheiden, durch einen Sputterprozess oder durch Chemical Vapor Deposition aufgebrachten, Clustern auf der Faser oder Oberfläche der Matrix vorliegt.

6. Wundauflage (10) nach einem der vorhergehenden Ansprüche, wobei die Substanz eine mittlere Partikelgröße von 5 bis 100 nm aufweist.

7. Wundauflage (10) nach einem der vorherigen Ansprüche, wobei die Substanz in einer Schicht mit einer mittleren Dicke von 5 bis 100 nm vorliegt.

8. Wundauflage (10) nach einem der vorhergehenden Ansprüche, wobei die Substanz in einer bei einem Durchnässen der Matrix (12) mit einer Flüssigkeit in der Matrix (12) antimikrobiell wirksamen Menge vorliegt.

9. Wundauflage (10) nach einem der vorhergehenden Ansprüche, wobei das Polymer ein die Adhäsion von Bakterien, vorzugsweise gramnegativen Bakterien oder Staphylokokken, insbesondere Staphylokokkus epidermidis, auf der Faser oder Matrix verminderndes Polymer ist.

10. Wundauflage (10) nach einem der vorhergehenden Ansprüche, wobei die Faser oder die Oberfläche der Matrix (12) eine mittels Plasmapolymerisation mit dem Polymer beschichtete Faser oder Oberfläche ist.

11. Wundauflage (10) nach Anspruch 10, wobei das Polymer ein nach der Plasmapolymerisation oxidiertes Polymer ist.

12. Wundauflage (10) nach einem der vorhergehenden Ansprüche, wobei das Polymer aus Monomeren auf Acrylsäurebasis oder aus Monomeren auf Siloxanbasis, insbesondere Hexamethyldisiloxan, gebildet ist.

13. Wundauflage (10) nach einem der vorherigen Ansprüche, wobei das Polymer in einer Schicht mit einer mittleren Dicke von 5 bis 500 nm vorliegt.

14. Wundauflage (10) nach einem der vorhergehenden Ansprüche, wobei die Substanz in einer Menge vorliegt, in der eine auf eine Wunde im Anwendungsfall nicht zytotoxisch wirkende Menge an Wirkstoffen von der Substanz gebildet und/oder freigesetzt wird.

15. Wundauflage (10) nach einem der vorhergehenden Ansprüche, wobei an der Matrix (12) eine Wundheilung unterstützende Substanzen, insbesondere Wachstumsfaktoren, gebunden sind.

16. Wundauflage (10) nach einem der vorhergehenden Ansprüche, wobei die Matrix (12) mit einer die Wundheilung unterstützenden, insbesondere sauren oder die Wundheilung unterstützende Nährstoffen enthaltenden, Flüssigkeit durchnäßt oder getränkt ist.

17. Wundauflage (10) nach einem der vorhergehenden Ansprüche, wobei die Folie (14), zumindest stellenweise, durchsichtig ist.

18. Wundauflage (10) nach einem der vorhergehenden Ansprüche, wobei die Wundauflage für Licht, insbesondere UV-Licht, IR-Licht oder NIR-Licht, durchlässig ist.

19. Wundauflage (10) nach einem der vorhergehenden Ansprüche, wobei die Wundauflage einen Indikator, insbesondere einen pH-Indikator, enthält.

20. Wundauflage (10) nach Anspruch 19, wobei der Indikator ein Sensor, insbesondere ein Biosensor, ist.

21. Wundauflage nach Anspruch 20, wobei der Sensor ein leitfähiges Polymer ist, welches in Abhängigkeit vom Zustand der Wunde oder wundauflage seine Leitfähigkeit ändert.

22. Wundauflage (10) nach einem der Ansprüche 19 bis 21, wobei der Indikator ein den Flüssigkeitsgehalt der Matrix (12) anzeigender Indikator ist.

23. Wundauflage (10) nach Anspruch 19 bis 21, wobei der Indikator ein den Grad und/oder Typ einer mikrobiellen Kontamination der Matrix (12) oder der Wunde anzeigender Indikator ist.

24. Wundauflage (10) nach Anspruch 19 bis 21, wobei der Indikator ein einen Entzündungsstatus der Wunde anzeigender Indikator ist.

25. Verfahren zur Herstellung einer Wundauflage (10) nach einem der vorhergehenden Ansprüche, mit folgenden Schritten:
- Bereitstellen einer saugfähigen Matrix (22),
- Bereitstellen einer gasdurchlässigen aber flüssigkeitsundurchlässigen Folie (14),
- Anbringen eines selbstklebenden ersten Bereichs (16) auf oder an der Folie (14) und
- Verbinden der saugfähigen Matrix (12) mit der Folie (14) in einem zweiten Bereich (17) der Folie (14),
- wobei der erste Bereich den zweiten Bereich (17) der Folie (14) umgibt,
wobei durch Verdampfen und Abscheiden, einen Sputterprozess oder Chemical Vapor Deposition zuerst eine antimikrobiell wirksame Substanz und dann durch Plasmapolymerisation ein Polymer auf der Matrix (12) oder einer die Matrix (12) bildenden Faser abgeschieden wird.

## Claims

1. A wound covering (10), comprising a first layer formed by an absorbent matrix (12) and an anti-microbially active substance, the substance being present chemically or physically bonded to one surface of the matrix (12), the surface of the matrix including the substance being coated with a hydrophilic polymer, the wound covering (10) comprising a second layer formed by a gas-permeable but liquid-impermeable film (14) connected to the matrix (12) and having a self-adhesive first area (16), the matrix (12) being connected to the film (14) in a second area (17) and the first area being an area of the film (14) which surrounds the second area (17), it being possible to stick the wound covering (10) to human or animal skin by means of the self-adhesive first area, a liquid-tight inner space which can be filled with a liquid comprising the matrix (12) being formed, the substance being a metal or a metal compound.

2. The wound covering (10) as claimed in claim 1, the matrix (12) having at least one fiber or being formed from at least one fiber and the surface of the matrix (12), to which the substance is bound, being a surface of the fiber.

3. The wound covering (10) as claimed in one of the preceding claims, the substance being bound exclusively to the surface of the matrix or fiber.

4. The wound covering (10) as claimed in one of the preceding claims, the substance being selected from a group consisting of silver, copper and zinc, or a mixture of silver, copper and zinc, or a mixture or alloy comprising at least one of these components.

5. The wound covering (10) as claimed in one of the preceding claims, the metal or the metal compound being present in the form of clusters on the fiber or surface of the matrix, in particular applied by evaporation and deposition, by a sputtering process or by chemical vapor deposition.

6. The wound covering (10) as claimed in one of the preceding claims, the substance having a mean particle size of 5 to 100 nm.

7. The wound covering (10) as claimed in one of the preceding claims, the substance being present in a layer having a mean thickness of 5 to 100 nm.

8. The wound covering (10) as claimed in one of the preceding claims, the substance being present in an amount which is antimicrobially active on thorough soaking of the matrix (12) with a liquid in the matrix (12).

9. The wound covering (10) as claimed in one of the preceding claims, the polymer being a polymer decreasing the adhesion of bacteria, preferably gram-negative bacteria or staphylococci, in particular Staphylococcus epidermidis, to the fiber or matrix.

10. The wound covering (10) as claimed in one of the preceding claims, the fiber or the surface of the matrix (12) being a fiber or surface coated with the polymer by means of plasma polymerization.

11. The wound covering (10) as claimed in claim 10, the polymer being a polymer oxidized after plasma polymerization.

12. The wound covering (10) as claimed in one of the preceding claims, the polymer being formed from monomers based on acrylic acid or from monomers based on siloxane, in particular hexamethyldisiloxane.

13. The wound covering (10) as claimed in one of the preceding claims, the polymer being present in a layer having a mean thickness of 5 to 500 nm.

14. The wound covering (10) as claimed in one of the preceding claims, the substance being present in an amount in which an amount of active compounds not acting cytotoxically on a wound in the application case is formed and/or released by the substance.

15. The wound covering (10) as claimed in one of the preceding claims, substances assisting wound healing, in particular growth factors, being bound to the matrix (12).

16. The wound covering (10) as claimed in one of the preceding claims, the matrix (12) being thoroughly soaked or impregnated with a liquid assisting wound healing, in particular an acidic liquid or a liquid comprising nutrients assisting wound healing.

17. The wound covering (10) as claimed in one of the preceding claims, the film (14) being transparent, at least in places.

18. The wound covering (10) as claimed in one of the preceding claims, the wound covering being transparent to light, in particular UV light, IR light or NIR light.

19. The wound covering (10) as claimed in one of the preceding claims, the wound covering comprising an indicator, in particular a pH indicator.

20. The wound covering (10) as claimed in claim 19, the indicator being a sensor, in particular a biosensor.

21. The wound covering as claimed in claim 20, the sensor being a conductive polymer which changes its conductivity depending on the state of the wound or wound covering.

22. The wound covering (10) as claimed in one of claims 19 to 21, the indicator being an indicator indicating the liquid content of the matrix (12).

23. The wound covering (10) as claimed in claim 19 to 21, the indicator being an indicator indicating the degree and/or type of a microbial contamination of the matrix (12) or of the wound.

24. The wound covering (10) as claimed in claim 19 to 21, the indicator being an indicator indicating an inflammatory status of the wound.

25. A process for the production of a wound covering (10) as claimed in one of the preceding claims, having the following steps:
- making available of an absorbent matrix (12),
- making available of a gas-permeable but liquid-impermeable film (14),
- application of a self-adhesive first area (16) on or to the film (14) and
- connection of the absorbent matrix (12) to the film in a second area (17) of the film (14),
- the first area surrounding the second area (17) of the film (14),
by evaporation and deposition, a sputtering process or chemical vapor deposition first an antimicrobially active substance and then, by plasma polymerization, a polymer being deposited on the matrix (12) or a fiber forming the matrix (12).

## Revendications

1. Pansement (10) contenant une première couche formée par une matrice absorbante (12) et une substance efficace sur le plan antimicrobien, dans lequel la substance se présente liée chimiquement ou physiquement à une surface de la matrice (12), dans lequel la surface de la matrice y compris la substance est revêtue d'un polymère hydrophile, dans lequel le pansement (10) contient une seconde couche liée à la matrice (12) et formée par une feuille (14) perméable au gaz mais imperméable au fluide et présente une première zone autocollante (16), dans lequel la matrice (12) est reliée avec la feuille (14) dans une seconde zone (17) et la première zone est une zone de la feuille (14) qui entoure la seconde zone (17), dans lequel le pansement (10) peut être collé au moyen de la première zone autocollante sur une peau humaine ou animale, dans lequel est formé un espace interne étanche au fluide contenant la matrice (12) et pouvant être rempli d'un fluide, et dans lequel la substance est un métal ou un composé métallique.

2. Pansement (10) selon la revendication 1, dans lequel la matrice (12) présente au moins une fibre ou est formée d'au moins une fibre, et la surface de la matrice (12) à laquelle est liée la substance est une surface de la fibre.

3. Pansement (10) selon l'une des revendications précédentes, dans lequel la substance est exclusivement liée au niveau de la surface de la matrice ou de la fibre.

4. Pansement (10) selon l'une des revendications précédentes, dans lequel la substance est sélectionnée dans un groupe composé de l'argent, du cuivre et du zinc, ou dans un mélange d'argent, de cuivre et de zinc, ou dans un mélange ou alliage comportant au moins un de ces composants.

5. Pansement (10) selon l'une des revendications précédentes, dans lequel le métal ou le composé métallique se présente sur la fibre ou la surface de la matrice sous la forme d'amas, appliqués en particulier par évaporation et dépôt, par un procédé de dépôt par pulvérisation ou par un dépôt chimique en phase vapeur.

6. Pansement (10) selon l'une des revendications précédentes, dans lequel la substance présente une taille de particules moyenne comprise entre 5 et 100 nm.

7. Pansement (10) selon l'une des revendications précédentes, dans lequel la substance se présente en une couche présentant une épaisseur moyenne comprise entre 5 et 100 nm.

8. Pansement (10) selon l'une des revendications précédentes, dans lequel la substance se présente dans la matrice (12) en une quantité efficace sur le plan antimicrobien avec une imbibition de la matrice (12) par un fluide.

9. Pansement (10) selon l'une des revendications précédentes, dans lequel le polymère est un polymère qui réduit l'adhésion des bactéries, de préférence des bactéries Gram négatives ou des staphylocoques, en particulier du staphylococcus epidermis, sur la fibre ou la matrice.

10. Pansement (10) selon l'une des revendications précédentes, dans lequel la fibre ou la surface de la matrice (12) est une fibre ou une surface revêtue du polymère au moyen d'une polymérisation au plasma.

11. Pansement (10) selon la revendication 10, dans lequel le polymère est un polymère oxydé après la polymérisation au plasma.

12. Pansement (10) selon l'une des revendications précédentes, dans lequel le polymère est formé de monomères à base d'acide acrylique ou de monomères à base de siloxane, en particulier d'hexaméthyldisiloxane.

13. Pansement (10) selon l'une des revendications précédentes, dans lequel le polymère se présente en une couche présentant une épaisseur moyenne comprise entre 5 et 500 nm.

14. Pansement (10) selon l'une des revendications précédentes, dans lequel la substance se présente en une quantité dans laquelle une quantité de principes actifs agissant sur une blessure en pratique de façon non cytotoxique est formée et/ou libérée par la substance.

15. Pansement (10) selon l'une des revendications précédentes, dans lequel sont liées à la matrice (12) des substances qui assistent une cicatrisation, en particulier des facteurs de croissance.

16. Pansement (10) selon l'une des revendications précédentes, dans lequel la matrice (12) est imbibée ou imprégnée d'un fluide qui assiste la cicatrisation, contenant en particulier des substances nutritives acides ou assistant la cicatrisation.

17. Pansement (10) selon l'une des revendications précédentes, dans lequel la feuille (14) est transparente, au moins par endroits.

18. Pansement (10) selon l'une des revendications précédentes, dans lequel le pansement est transparent à la lumière, en particulier à la lumière UV, à la lumière IR ou à la lumière NIR (spectre du proche infrarouge).

19. Pansement (10) selon l'une des revendications précédentes, dans lequel le pansement contient un indicateur, en particulier un indicateur de pH.

20. Pansement (10) selon la revendication 19, dans lequel l'indicateur est un capteur, en particulier un capteur biologique.

21. Pansement selon la revendication 20, dans lequel le capteur est un polymère conducteur qui modifie sa conductibilité en fonction de l'état de la blessure ou du pansement.

22. Pansement (10) selon l'une des revendications 19 à 21, dans lequel l'indicateur est un indicateur qui montre la teneur en fluide de la matrice (12).

23. Pansement (10) selon la revendication 19 à 21, dans lequel l'indicateur est un indicateur qui montre le degré et/ou le type d'une contamination microbienne de la matrice (12) ou de la blessure.

24. Pansement (10) selon la revendication 19 à 21, dans lequel l'indicateur est un indicateur qui montre un état d'inflammation de la blessure.

25. Procédé de fabrication d'un pansement (10) selon l'une des revendications précédentes, comportant les étapes suivantes :
- mise à disposition d'une matrice absorbante (12) ;
- mise à disposition d'une feuille (14) perméable au gaz mais imperméable au fluide ;
- application d'une première zone autocollante (16) sur ou contre la feuille (14) ; et
- liaison de la matrice absorbante (12) avec la feuille (14) dans une seconde zone (17) de la feuille (14) ;
- où la première zone entoure la seconde zone (17) de la feuille (14),
dans lequel par évaporation et dépôt, un procédé de dépôt par pulvérisation ou un dépôt chimique en phase vapeur, on dépose d'abord sur la matrice (12) ou une fibre formant la matrice (12) une substance efficace sur le plan antimicrobien puis un polymère par polymérisation au plasma.
